# EUROPÄISCHE PATENTSCHRIFT

(11) **EP 2 586 868 B1**
(45) Veröffentlichungstag und Bekanntmachung des Hinweises auf die Patenterteilung: **22.05.2019**
(21) Anmeldenummer: 12007275.6
(22) Anmeldetag: 22.10.2012
(51) Int. Cl.: C12P 5/02, C12M 1/107

(54) **Verfahren und Biogasanlage zum Erzeugen von Biogas**
Method and biogas system for the generation of biogas
Procédé et installation de biogaz pour la production de biogaz

(30) Priorität: 25.10.2011 DE 102011116843
(43) Veröffentlichungstag der Anmeldung: 01.05.2013
(73) Patentinhaber: E.ON Bioerdgas GmbH, 45131 Essen (DE)
(72) Erfinder: Freiherr von Canstein, Harald, 40567 Meerbusch (DE); Beining, Jean Relus, 44388 Dortmund (DE); Vogel, Alexander, 40885 Ratingen (DE); Adelt, Marius, 40286 Dorsten (DE)
(74) Vertreter: Harlacher, Mechthild

(56) Entgegenhaltungen:
- EP-A1- 1 811 015
- EP-A1- 1 818 315
- WO-A1-2010/014919
- DE-A1-102008 033 049
- DE-A1-102009 018 126
- DE-A1-102010 043 630

## Beschreibung

Die Erfindung betrifft ein Verfahren zum Erzeugen von Biogas in einer Biogasanlage mit den Schritten,
a) Vergären einer Biomasse in mindestens einem ersten Fermentationsbehälter während einer ersten Gärstufe unter Bildung eines Fermentationsrückstandes und eines Rohbiogases,
b) Austragen des Fermentationsrückstandes aus dem ersten Fermentationsbehälter und Eintragen des Fermentationsrückstandes in mindestens einen weiteren Fermentationsbehälter zum Nachgären des Fermentationsrückstandes in einer zweiten und/oder weiteren Gärstufe zum Erzeugen von Biogas in einer Biogasanlage wobei eine Biomasse in mindestens einem Fermentationsbehälter während einer ersten Gärstufe unter Bildung eines Fermentationsrückstandes und eines Rohbiogases vergoren wird.

Ferner betrifft die Erfindung eine Biogasanlage zum Erzeugen von Biogas in einer Biogasanlage, mit mindestens einem Fermentationsbehälter, in dem Biomasse während einer ersten Gärstufe unter Bildung eines Fermentationsrückstandes und eines Rohbiogases vergoren wird und mit mindestens einem weiteren Fermentationsbehälter zum Nachgären des Fermentationsrückstandes in einer zweiten und/oder weiteren Gärstufe.

Aus der DE 10 2010 043 630 A1 ist ein Verfahren, eine Anlage und ein Methanreaktor zur Erhöhung der Methankonzentration des Biogases aus Biogasanlagen bekannt. In der zweistufigen Biomasse-Vergärungsanlage wird in einer Hydrolysestufe in einem Hydrolysereaktor ein Hydrolysegas gebildet, welches überwiegend aus Wasserstoff (H₂) und Kohlenstoffdioxid (CO₂) besteht. Das entstehende Hydrolysegas wird aufgetrennt und der hieraus erhaltene Wasserstoff in einen Methanreaktor mit Ober- und Untersäule eingeleitet. Alternativ kann der Wasserstoff aus einer externen Quelle stammen. Die Einleitung des Wasserstoffs erfolgt in der Obersäule des Methanreaktors, die im Stoffaustausch mit dem eigentlichen Fermenter in der Untersäule des Methanreaktors steht. In der Untersäule befinden sich immobilisierte acetotrophe Methanbildner und in der Obersäule suspendierte/immobilisierte hydrogenotrophe Methanbildner. Aus der Untersäule strömt das dort gebildete Biogas einschließlich seines CO₂-Gehaltes nach oben in die Obersäule, wo das CO₂ mit zugeführten Wasserstoff von den hydrogenotrophen Methanbildnern zu Methan verstoffwechselt wird.

Ferner ist aus der DE 10 2009 053 593 A1 ein Verfahren und eine Vorrichtung zum Wasserstofftransfer in einen Methanfermenter bekannt. Wasserstoff, der mittels Elektrolyse aus Wasser generiert worden ist, wird unter Druck einem Wasserstoff-Metallhydridspeicher in einem gasdichten Behälter zugeführt und gespeichert. In Abhängigkeit von den physikalischen Umgebungsparametern (Gärungstemperatur u. dgl.) wird atomarer Wasserstoff über eine Antibiofilm-Membrane an Biomasse abgegeben, die sich in einem Methanfermenter befindet. In dem Methanfermenter wird in einem einstufigen Verfahren aus dem Wasserstoff und dem im Methanfermenter entstehenden Kohlendioxids Methan erzeugt. Die H2-Zufuhr zu der Biomasse ist nicht regelbar und die Methanausbeute begrenzt.

Die Erzeugung von Biogas aus Biomasse bzw. biologisch abbaubaren Rohstoffen wie insbesondere nachwachsenden Rohstoffen, ist eine der wesentlichen Technologien der regenerativen Energieerzeugung.

In der Praxis sind die Mehrzahl der Biogasanlagen sogenannte Direktverstromungsanlagen, in denen das Biogas in einem Blockheizkraftwerk, welches sich in räumlicher Nähe zur Biogasanlage befindet, in Strom und Wärme umgewandelt wird.

Als Alternative werden zunehmend Bio-Erdgas-Anlagen (auch Biomethan-Anlagen genannt) gebaut, bei denen das Biogas einer Aufbereitungsanlage zugeführt und durch Entfernen von CO₂ in Bio-Erdgas umgewandelt wird, insbesondere um das Bio-Erdgas in ein öffentliches Erdgasversorgungsnetz einzuspeisen. Für die Aufbereitung von Biogas zu Bio-Erdgas existieren verschiedene Technologien. Den gängigen Technologien wie Druckwasserwäsche, Aminwäsche, Selexolwäsche, Druckwechseladsorption und Membranverfahren ist gemein, dass das CH₄ im Gasstrom verbleibt und das CO₂ entfernt wird. Nachteilig ist, dass die Aufbereitungskapazität der Verfahren durch den Massenstrom an CO₂ begrenzt sind.

Aufgrund des deutschen Gesetzes für den "Vorrang Erneuerbarer Energien" (Erneuerbare-Energien-Gesetz, EE-Gesetz) erfolgt in Deutschland neben der Biogaserzeugung ein sehr schneller Ausbau der erneuerbaren Stromerzeugung (EE-Strom), insbesondere aus Wind und Photovoltaik. Diese Erzeugung ist stark fluktuierend und vom momentanen Bedarf unabhängig, was zeitweise zu Stromüberschüssen im Netz und wegen begrenzter Stromspeicherungsmöglichkeiten zu zwangsläufiger Anlagenabschaltung führt. Da das deutsche EE-Gesetz von der Mehrzahl von Staaten in der Europäischen Union als Vorbild für eigene Förderinstrumente herangezogen wurde, stellt sich das Problem auch in anderen Staaten.

Da sich dieses Problem mit dem steigenden Anteil des EE-Stroms im Netz verschärft, wird die Nutzung der Stromüberschüsse zur Wasserstofferzeugung durch Wasserelektrolyse als eine Option mit hohem Potenzial gehandelt. Der Wasserstoff kann aber nur zu einem gewissen Anteil dem Erdgas in einem öffentlichen Erdgasversorgungnetz beigemischt werden.

Vor dem oben beschriebenen Hintergrund liegt der Erfindung die Aufgabe zugrunde, ein Verfahren und eine Biogasanlage der eingangs genannten Art so zu optimieren, dass regenerativ erzeugte Energie optimal nutzbar wird.

Die gestellte Aufgabe wird durch die Verfahren mit den Merkmalen des Anspruchs 1 gelöst.

Bei einem Verfahren der eingangs genannten Art wird erfindungsgemäß Wasserstoff, der mittels Elektrolyse aus Wasser und Strom erzeugt wird und ein CO₂-haltiges Gas, wobei als CO₂-haltiges Gas Rohbiogas verwendet wird, in den Fermentationsrückstand während der zweiten und/oder weiteren Gärstufe eingeleitet und biologisch Wasserstoff und CO₂ zu Methan umgewandelt.

Die Erfindung beruht auf der Erkenntnis, dass in der ersten Gärstufe die Biomasse in dem ersten Fermentationsbehälter durch die Mikroorganismen weitgehend abgebaut wird. In dem Fermentationsrückstand, der in einem weiteren Fermentationsbehälter während einer weiteren Gärstufen weiter vergärt, arbeiten die Mikroorganismen unterhalb ihrer Leistungskapazität und produzieren pro Faulraumvolumen zunehmend weniger Methan. Versuche haben gezeigt, dass die Aktivität der Mikroorganismen, insbesondere die Aktivität der methanogenen Archaeen ausreichend hoch ist, um aus dem in den Fermentationsrückstand eingeleiteten Wasserstoff mittels des CO₂-haltigen Gases Methan zu bilden.

Ganz besonders vorteilhaft ist, dass in der zweiten Gärstufe in dem weiteren Fermentationsbehälter keine Nährstoffe zugegeben werden müssen, weil kein Mangel an mikrobieller Aktivität zu befürchten ist. Der erste Fermentationsbehälter als Hauptfermenter wird kontinuierlich mit Biomasse bzw. Substraten und bei Bedarf mit Spurenelementen versorgt, so dass kontinuierlich hoch aktive und mit Nährstoffen wohl versorgte Archaeen bzw. Mikroorganismen aus dem ersten Fermentationsbehälter bzw. dem Hauptfermenter in die weiteren, insbesondere den zweiten Fermentationsbehälter ausgetragen werden.

Das auf erfindungsgemäße Art erzeugte methanhaltige Biogas weist einen geringeren Kohlenstoffdioxid-Anteil auf als herkömmliche, aus der gleichen Biomasse erzeugte, Biogase. Dadurch wird die Aufbereitung von Biogas zu Bio-Erdgas vereinfacht und begünstigt.

Vorzugsweise wird als CO₂-haltiges Gas Rohbiogas verwendet, vorzugsweise Rohbiogas aus der ersten Gärstufe.

Nach der Erfindung wird der Wasserstoff mittels Elektrolyse aus Wasser und Strom erzeugt. Der Wasserstoff wird vorzugsweise mittels Wasserelektrolyse aus Strom aus erneuerbaren Quellen erzeugt. Bevorzugt wird Strom aus Windkraftanlagen zur Erzeugung von Wasserstoff genutzt. Der Strom wird je nach Anfall in Wasserstoff umgewandelt. Der jeweils anfallende Wasserstoff wird in die Biogasanlage geleitet und im Rahmen der Erfindung in Methan umgewandelt. Alternativ kann der Wasserstoff mittels thermochemischer Behandlung von Biomasse oder mittels Photokatalyse erzeugt werden.

Vorzugsweise werden der Wasserstoff und das Rohbiogas derart in den Fermentationsrückstand eingeleitet, dass der Wasserstoff und das Rohbiogas durch den Fermentationsrückstand als feine Blasen strömt, um eine möglichst hohe Grenzfläche für den Übertritt der Gase in das Gärmedium zu schaffen.

Der Wasserstoff und das CO₂-haltige Gas werden vorzugsweise gleichzeitig in den Fermentationsrückstand eingeleitet. Der Wasserstoff und Rohbiogas werden erfindungsgemäß gemischt und als Gasgemisch in den Fermentationsrückstand eingeleitet.

Erfindungsgemäß werden der Wasserstoff und das Rohbiogas mittels mindestens einer sich wenigstens teilweise über den Querschnitt des Fermentationsbehälters erstreckenden Zuführung in den Fermentationsrückstand eingeleitet.

Bei einer bevorzugten Ausführungsform der Erfindung wird das Rohbiogas in Abhängigkeit von dessen Wasserstoffgehalt in einer Kaskade in den Fermentationsrückstand in mindestens einem weiteren Fermentationsbehälter während der zweiten und/oder weiteren Gärstufe geleitet.

Im Rahmen der Erfindung wird das Rohbiogas am Ende der zweiten oder weiteren Gärstufe einer Aufbereitungsanlage zugeführt und mittels Entfernung von CO₂ in Bio-Erdgas umgewandelt.

Das Bio-Erdgas kann im Rahmen der Erfindung in ein öffentliches Erdgasversorgungsnetz eingespeist und in dem Netz oder in untertägigen Speichern unbegrenzt gespeichert werden. Folglich ermöglicht die Erfindung die unbegrenzte Speicherung von Strom aus erneuerbaren Quellen.

Alternativ kann das erfindungsgemäß CH₄-reiche Bio-Erdgas gespeichert, bedarfsweise ausgespeichert und einer Verstromungsanlage zugeführt werden. Da das Bio-Erdgas aufgrund des hohen CH₄ -Gehaltes einen hohen Energieinhalt aufweist, kann bei gegebenem Gasspeichervolumen in einer Direktverstromungsanlage eine höhere Energiemenge gespeichert und ggf. zeitverzögert und bedarfsgerecht verstromt werden.

Die Erfindung beinhaltet ferner eine Biogasanlage zum Erzeugen von Biogas mit mindestens einem ersten Fermentationsbehälter, in dem Biomasse während einer ersten Gärstufe unter Bildung eines Fermentationsrückstandes und eines Rohbiogases vergoren wird, mit mindestens einem weiteren Fermentationsbehälter zum Nachgären des Fermentationsrückstandes in einer zweiten und/oder weiteren Gärstufe und mit mindestens einer Zuführung, die eintrittsseitig an eine Wasserstoffleitung und eine Leitung für CO₂-haltiges Gas angeschlossen ist, wobei die Wasserstoffleitung an eine Wasserelektrolyse angeschlossen ist, wobei durch die Zuführung ausgangsseitig Wasserstoff und Rohbiogas in den Fermentationsrückstand in der zweiten und/oder weiteren Gärstufe eingeleitet wird, wobei der Wasserstoff mittels Kohlenstoffdioxid biologisch zu Methan umgewandelt wird.

In der für die Methanisierung vorgesehenen zweiten und/oder weiteren Gärstufe in dem zweiten und/oder weiteren Fermentationsbehälter weicht die Temperatur nur um wenige Grad Celsius von der Temperatur der vorhergehenden Gärstufe ab. Eine Abweichung von mehreren Grad Celsius nach unten wie nach oben würde die Aktivität der beteiligten Mikroorganismen reduzieren. Eine ausreichend dicke Isolierung des weiteren Fermentationsbehälters reicht in der Regel aus, eine hinreichend homogene Aktivität der Mikroorganismen zu gewährleisten.

Vorteilhafterweise erstreckt sich die Zuführung in Bodennähe des Fermentationsbehälters in dem die Nachgärung des Fermentationsrückstandes in einer zweiten und/oder weiteren Gärstufe stattfindet, so dass der Wasserstoff und CO₂-haltiges Gas durch den Fermentationsrückstand von unten nach oben strömen. Die Zuführung kann beispielsweise seitlich durch die Wand des Fermentationsbehälters geführt werden.

Bei einer bevorzugten Weiterbildung der Erfindung ist die Zuführung als mindestens eine Lanze ausgebildet, die sich wenigstens teilweise über den Querschnitt des Fermentationsbehälters erstreckt. Mehrere Lanzen können mittels einer ringförmigen Leitung an die Wasserstoffleitung und die Leitung für CO₂-haltiges Gas angeschlossen werden.

Alternativ kann die Zuführung als mindestens eine konzentrische Ringleitung oder als Hohlboden des Fermentationsbehälters ausgebildet werden.

Erfindungsgemäß kann die Zuführung eine Vielzahl von Durchtrittsöffnungen in Form einer Gitter- oder Lochstruktur aufweisen. Vorzugsweise weist die Zuführung einen Schieber zum Entfernen von Sedimenten von den Durchtrittsöffhungen auf.

Im Rahmen der Erfindung kann die Zuführung wenigstens teilweise als Membran ausgebildet sein.

Die Erfindung wird im Folgenden anhand eines bevorzugten Ausführungsbeispiels einer erfindungsgemäßen Anlage zum Erzeugen von Biogas im Zusammenhang mit der Zeichnung näher erläutert.

Die Zeichnung zeigt in:
Fig. 1 eine schematische Darstellung einer Anlage zum Erzeugen von Biogas nach dem Stand der Technik;
Fig. 2 eine schematische Darstellung einer erfindungsgemäßen Anlage zum Erzeugen von Biogas;
Fig. 3 eine schematische Draufsicht auf einen Fermentationsbehälter 2 in einer Anlage nach Fig. 2 in einer ersten Ausführungsform;
Fig.4 eine schematische Draufsicht auf einen Fermentationsbehälter 2 in einer Anlage nach Fig. 2 in einer zweiten Ausführungsform;
Fig. 5 eine schematische Draufsicht auf einen Fermentationsbehälter 2 in einer Anlage nach Fig. 2 in einer dritten Ausführungsform;
Fig. 6 eine schematische Draufsicht auf einen Fermentationsbehälter 2 in einer Anlage nach Fig. 2 in einer vierten Ausführungsform.

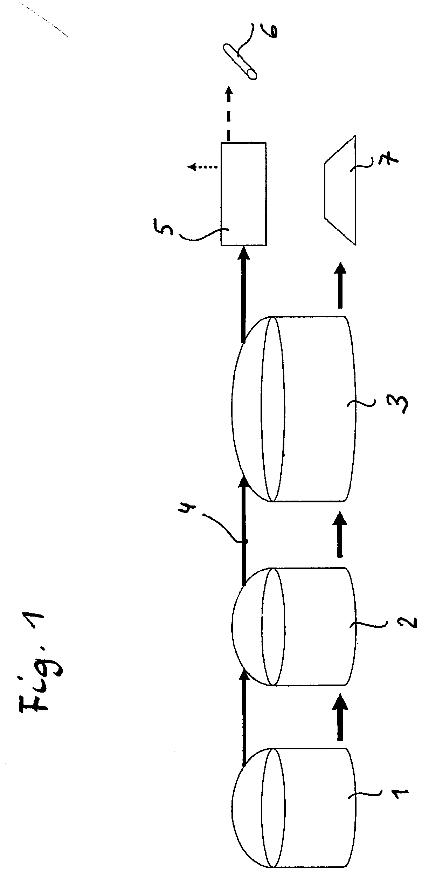

Die in Fig. 1 schematisch dargestellte Anlage zum Erzeugen von Biogas nach dem Stand der Technik weist einen ersten Fermentationsbehälter 1, einen zweiten Fermentationsbehälter 2 in Form eines Nachgärers und einen dritten Fermentationsbehälter 3 in Form eines Gärrestlagers auf. In derartigen Biogasanlagen mit mehreren Gärstufen wird die Biomasse in der Regel in den Fermentationsbehälter 1 eingetragen, dort in eine ersten Gärstufe weitgehend zu Rohbiogas vergoren und der dabei entstehende Fermentationsrückstand nach Verweilzeiten von Tagen bis Monaten ausgetragen und anschließend in den Nachgärer 2 eingetragen und weiter vergoren. Nach Verweilzeiten von erneut Tagen bis Monaten wird der Fermentationsrückstand aus dem Nachgärer 2 in das Gärrestlager 3 für eine dritte Gärstufe eingetragen.

Beim Vergären der Biomasse in der ersten Gärstufe in dem ersten Fermentationsbehälter 1 ist die Methanbildungsrate hoch und nimmt beim Nachgären des Fermentationsrückstandes in den weiteren Fermentationsbehältern 2, 3 während der weiteren Gärstufen immer weiter ab. Die Methanbildungsrate hängt insbesondere von der Verdaulichkeit der jeweiligen Biomasse bzw. der Substrate, der Temperatur und der organischen Raumbelastung ab.

Bei Versuchen wurden in der ersten Gärstufe im ersten Fermentationsbehälter 1 Raten von 0,06 bis 0,13 Nm³ CH₄ pro m³ Faulraum und Stunde ermittelt. Die Methanbildungsraten im zweiten Fermentationsbehälter 2 in der zweiten Gärstufe betrugen ca. 20 % der Methanbildungsraten im ersten Fermentationsbehälter 1 während der ersten Gärstufe und nur noch 1 % in der dritten Gärstufe im Gärrestlager 3.

Die aus der Praxis bekannten Fermentationsbehälter 1 bis 3 sind topfförmig und haben einen runden oder quadratischen Querschnitt. Die Fermentationsbehälter werden aus Beton oder Stahlplatten gasdicht ausgebildet. Der Durchmesser kann ca. 20 m betragen. Die Höhe der Fermentationsbehälter kann ca. 10 m betragen. In dem ersten Fermentationsbehälter 1 befindet sich in der Regel ein nicht dargestelltes Rührwerk. Bei dem Fermentationsbehälter 2 handelt es sich um einen Nachgärer. Der Fermentationsbehälter 3 ist als Gärlager ausgebildet. Alle drei Fermentationsbehälter sind weitgehend mit der Biomasse bzw. dem Fermentationsrückstand gefüllt. Im oberen Bereich wird ein Gasraum gebildet, in dem sich das entstehende Rohbiogas sammelt. Bei der aus dem Stand der Technik bekannten Anlage sind die Gasräume der hintereinander angeordneten Fermentationsbehälter über eine Biogasleitung 4 miteinander verbunden. Aus der letzten Gärstufe im Fermentationsbehälter 3 wird das Biogas einer Aufbereitungsanlage 5 zugeführt und durch Entfernung von insbesondere CO₂ zu Bio-Erdgas umgewandelt, welches in ein öffentliches Erdgasversorgungsnetz 6 eingeleitet wird. Der Fermentationsrückstand kann auf einen Acker 7 ausgebracht werden.

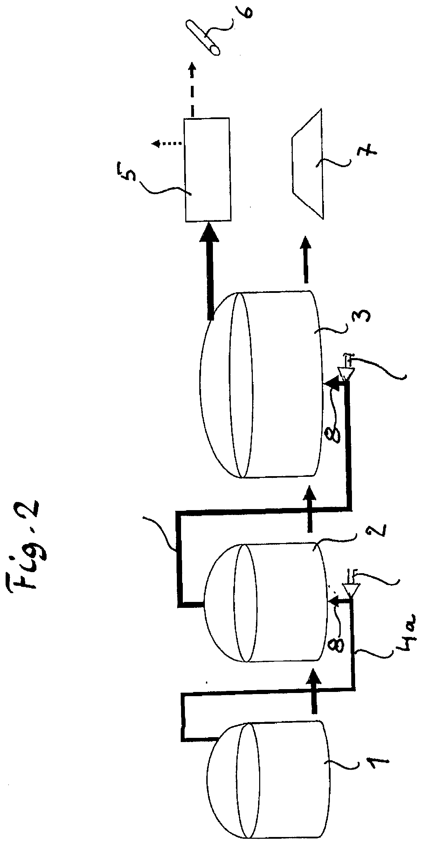

Bei der in Fig. 2 dargestellten erfindungsgemäßen Anlage weist - wie die Anlage nach Fig. 1 - drei Fermentationsbehälter 1, 2 und 3 auf. Wie bei der Anlage nach Fig. 1 wird die Biomasse in den Fermentationsbehälter 1 eingetragen, dort in eine ersten Gärstufe weitgehend zu Rohbiogas vergoren und der dabei entstehende Fermentationsrückstand nach Verweilzeiten von Tagen bis Monaten ausgetragen und in den Fermentationsbehälter 2 eingetragen und weiter vergoren. Nach Verweilzeiten von erneut Tagen bis Monaten wird der Fermentationsrückstand aus dem Fermentationsbehälter 2 ausgetragen und in den Fermentationsbehälter 3 für eine dritte Gärstufe eingetragen. An dem zweiten und dem dritten Fermentationsbehälter 2, 3 ist jeweils schematisch eine Zuführung 8 dargestellt, an die eintrittseitig eine Biogasleitung 4a und eine Wasserstoffleitung 9 angeschlossen sind, so dass Wasserstoff und CO₂-haltiges Gas in den Fermentationsrückstand in den Fermentationsbehältern 2, 3 während der zweiten und dritten Gärstufe eingeleitet werden können. Wichtig ist, dass beide Gase gleichzeitig in den Fermentationsrückstand eingeleitet werden.

Die Zuführung 8 ist in der Nähe des Bodens der Fermentationsbehälter 2, 3 angeordnet. Die Biogasleitung 4a ist jeweils an den Fermentationsbehälter in Höhe des Gasraumes angeschlossen, in dem die vorangegangene Gärstufe stattgefunden hat. Folglich ist die Biogasleitung 4a einerseits an die Zuführung im zweiten Fermentationsbehälter 2 und anderseits an den Gasraum des ersten Fermentationsbehälters 1 angeschlossen, so dass Rohbiogas aus dem ersten Fermentationsbehälter 1 in die Zuführung 8 in dem zweiten Fermentationsbehälter 2 strömt. Von dem zweiten Fermentationsbehälter 2 führt die Biogasleitung 4a von dem Gasraum des zweiten Fermentationsbehälters 2, an dem sie angeschlossen ist, zu der Zuführung 8 am dritten Fermentationsbehälter 3.

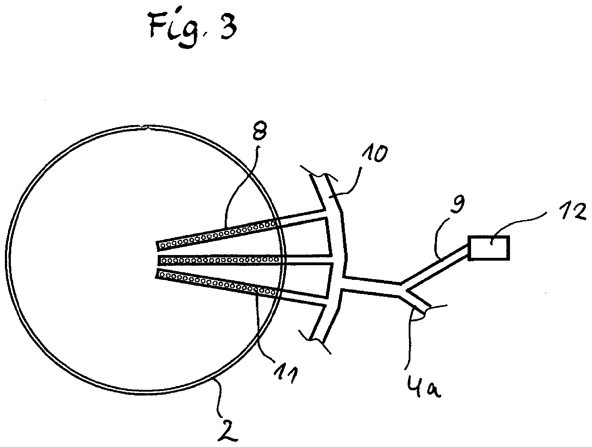

In Fig. 3 ist dargestellt, dass die Zuführung 8 in Form von mehreren Lanzen ausgebildet ist. Jede Lanze ist eintrittsseitig mittels einer Ringleitung 10 an die Biogasleitung 4a und die Wasserstoffleitung 9 angeschlossen. Das Wasserstoff- und das CO₂- haltige Gasgemisch strömt durch eine Vielzahl von nach oben gerichteten Durchtrittsöffhungen 11 von unten in den Fermentationsrückstand in dem Fermentationsbehälter 2. In den Fig. 3 bis 6 ist jeweils der Fermentationsbehälter 2 dargestellt. Der Fermentationsbehälter 3 ist entsprechend ausgerüstet.

Die Lanze 8 erstreckt sich in Bodennähe teilweise über den Querschnitt des Fermentierungsbehälters 2. Die Lanzen 8 könnten auch unterschiedlich lang sein und sich im Wesentlichen vollständig über den Querschnitt des Fermentationsbehälters 2 erstrecken.

Der Durchtrittsquerschnitt der einzelnen Durchtrittsöffhungen 10 und der Gasvolumenstrom des Gasgemisches sind so zu wählen, dass das Gasgemisch in möglichst kleinen Blasen austritt, um eine möglichst große Kontaktfläche zum Fermentationsrückstand bzw. Gärmedium zu erreichen.

Der Fermentationsrückstand ist weitgehend flüssig. Der Druck des Gasgemisches sollte so gering als möglich sein und ist vorzugsweise gerade so hoch um den hydrostatischen Druck zu überwinden.

Der zweite Fermentationsbehälter 2 weist eine nicht dargestellte Isolierung oder Heizung auf, so dass gewährleistet ist, dass die Gärtemperatur nicht oder nur um wenige Grad Celsius von der Gärtemperatur des vorherigen Gärbehälters abweicht.

Die Konzentration von H₂ und CO₂ in dem zugeführten Gasgemisch steht mit der entsprechenden Konzentration von H₂ und CO₂ im Fermentationsrückstand bzw. im Gärmedium im Gleichgewicht. Durch die Aufnahme von H2 und CO₂ in die Zellen der Mikroorganismen und die Umwandlung in CH₄ entsteht ein Konzentrationsgefälle, so dass weiteres H₂ und CO₂ in den Fermentationsrückstand in einer Menge eingeleitet wird.

Dabei muss beachtet werden, dass in dem Fermentationsbehälter 2 selbst noch CO₂ gebildet wird und nennenswerte Mengen sowohl chemisch als Hydrogenkarbonat- und Karbonat-Ionen als auch physikalisch als Gas vorliegen. Ein Mangel an CO₂ resultiert in einer unvollständigen Methanisierung des H2 und Anreicherung von H2 im Biogas.

Die Methanisierung erfolgt nach folgender Reaktionsgleichung:

4 H₂ + 1 CO₂ → 1 CH₄ + 2 H₂O

Da Methan nur eine geringe Wasserlöslichkeit hat, sammelt es sich in Gasbläschen, steigt auf und wird im Gasraum der Gärbehälter bzw. Fermentationsbehälter aufgefangen. Dies wird durch eine geringe Viskosität des Fermentationsrückstandes bzw. des Gärmediums, z. B. durch die Entfernung von Fasern durch Separation, begünstigt.

Das Wasser verbleibt im Gärmedium und erhöht dort leicht den Wassergehalt, was sich positiv auf die Viskosität und den Massentransfer auswirkt.

Die Bedingungen im zweiten Fermentationsbehälter 2, insbesondere Belastung mit unverdauter Biomasse, Temperatur, Viskosität, Wassergehalt, Vermischung, Höhe des Fermentationsrückstandes bzw. des Gärmediums, bedingen die Effizienz der Umsetzung. Unter optimalen Bedingungen kann im Fermentationsbehälter 2 die komplette Menge CO₂ des Rohbiogases mit einer entsprechenden Menge H2 zu CH₄ umgewandelt werden.

Die Wasserstoffleitung 9 ist an eine in den Fig. 3 bis 6 schematisch dargestellte Wasserelektrolyse 12 angeschlossen, in welcher Wasserstoff mittels Strom aus einem nicht dargestellten Windkraftwerk erzeugt wird.

Bei einer unvollständigen Methanisierung kann das Rohbiogas, welches Restmengen an H2 und CO₂ aufweist, in einer Kaskade durch einen oder mehrere weitere Nachgärer und/oder Gärrestlager geführt werden. Zur verbesserten Umsetzung von H2 kann ferner eine Rezirkulation des Biogases in eine der Gärstufen vorgesehen werden. Die Rezirkulationsrate wird in diesem Fall über den H₂-Gehalt im Rohbiogas gesteuert. Aus der letzten Gärstufe im Fermentationsbehälter 3 wird das Biogas einer Aufbereitungsanlage 5 zugeführt und durch Entfernung von insbesondere CO₂ zu Bio-Erdgas umgewandelt, welches in ein öffentliches Erdgasversorgungsnetz 6 eingeleitet wird. Der Fermentationsrückstand kann auf einen Acker 7 ausgebracht werden.

Der Vorteil des hier vorgestellten Verfahrens ist die Umwandlung von CO₂ in CH₄, so dass bei konstantem Volumenstrom der CO₂-Volumenstrom auf bis zu Null reduziert und der Methan-Massenstrom auf bis das Doppelte erhöht werden kann. Dadurch kann bei deutlich reduzierten Aufbereitungskosten eine deutlich größere Menge Bio-Erdgas eingespeist werden.

Die Eignung verschiedener Biogas-Aufbereitungsverfahren für das vorgeschlagene Verfahren ist unterschiedlich. So sind die Membranen, welche für die Biogasaufbereitung mittels Membranverfahren eingesetzt werden, i. d. R. sehr durchlässig für H₂, welches in geringen Anteilen noch im erzeugten Gas vorhanden sein kann. Dies führt bei konventioneller Anlagenkonfiguration dazu, dass H₂ überwiegend in den CO₂ -reichen Abgasstrom überführt wird. Bei anderen Verfahren, wie z. B. Aminwäschen, würde H₂ voraussichtlich überwiegend in das Produktgas überführt werden. Der Vorteil der überwiegenden Überführung des verbleibenden H₂ in das Produktgas ist der zusätzliche Energiegehalt im Proist die jeweilige Beschränkung des maximalen H₂ -Anteils im Produktgas. Je nach zulässigem H₂ -Gehalt im erzeugten Gas können sich somit jeweils unterschiedliche Gasaufbereitungsverfahren besonders gut zur Kombination mit dem vorgeschlagenen Verfahren eignen.

Die Biogaserzeugung wird nicht wie - beim Stand der Technik - durch die Gasaufbereitungstechnik begrenzt, denn bei konstantem Volumenstrom kann der CO₂-Volumenstrom auf bis zu Null reduziert und der Methan-Massenstrom auf das Doppelte erhöht werden.

Nicht umgesetzter Wasserstoff kann in das Bio-Erdgas eingespeist werden. Die Höchstmenge ergibt sich aus den Regelwerken DVGW 260 und DVGW 2062 von 2007, die der Gasnetzzugangsverordnung zugrunde liegen. Es gibt keinen festen Grenzwert, sondern die maximale Wasserstoffkonzentration ergibt sich im Einzelfall aus den im jeweiligen Netzgebiet vorgegebenen Kennwerten Wobbe-Index und Brennwert.

Das erfindungsgemäße Verfahren dient dazu, den so genannten Überschussstrom in Bio-Erdgas und somit in eine speicherbare Energieform zu überführen, die Kosten der Bio-Erdgas-Einspeisung zu reduzieren, das Stromnetz zu entlasten und somit den weiteren Ausbau von erneuerbare Energie-Anlagen zu ermöglichen.

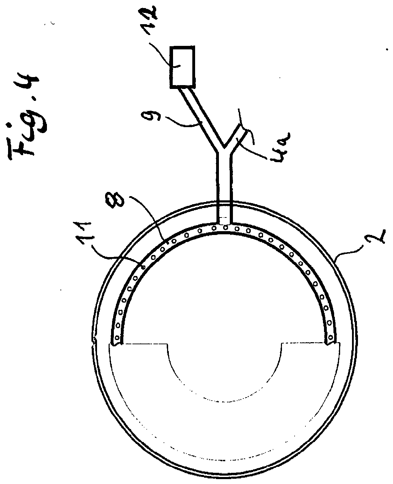

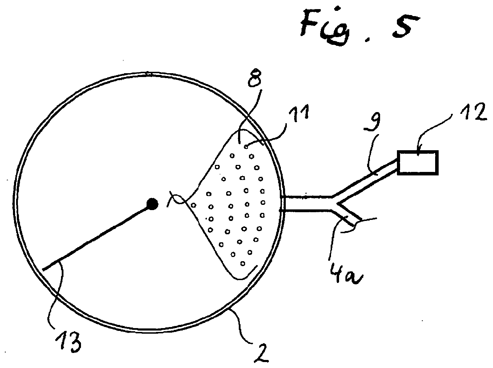

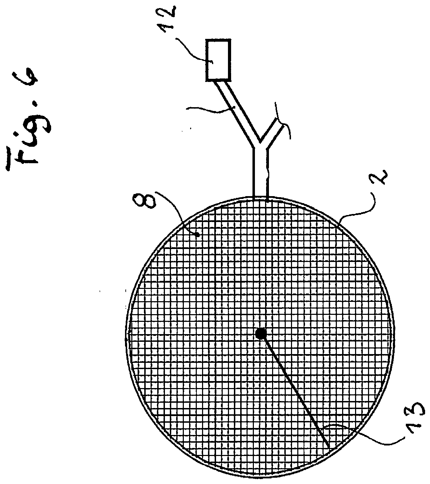

Im Rahmen der Erfindung kann, wie in Fig. 4 dargestellt, die Zuführung 8 als Ringleitung mit einer Vielzahl von Durchtrittsöffhungen 11 ausgeführt werden. In den Fig. 5 und 6 ist die Zuführung als unterströmte Bodenplatte ausgeführt, die zudem mit einem Schieber 13 zur Entfernung von Ablagerungen ausgerüstet werden kann.

Die Durchtrittsöffhungen 11 sind kreis- oder schlitzförmig. Sie können jede beliebige Form aufweisen, die gewährleistet, dass Wasserstoff und das CO₂-haltige Gas in einer Vielzahl von feinen Blasen durch den Fermentationsbehälter strömt. Alternativ kann die Zuführung 8 anstelle der Durchtrittsöffhungen eine Membran 11 aufweisen.

**Bezugszeichenliste**

| | |
|---|---|
| 1 | Erster Fermentationsbehälter |
| 2 | Zweiter Fermentationsbehälter |
| 3 | Dritter Fermentationsbehälter |
| 4 | Biogasleitung |
| 4a | Biogasleitung |
| 5 | Aufbereitungsanlage |
| 6 | Erdgasversorgungsnetz |
| 7 | Acker |
| 8 | Zuführung |
| 9 | Wasserstoffleitung |
| 10 | Ringleitung |
| 11 | Durchtrittsöffnungen |
| 12 | Wasserelektrolyse |
| 13 | Schieber |

## Patentansprüche

1. Verfahren zum Erzeugen von Biogas in einer Biogasanlage mit den Schritten
a) Vergären einer Biomasse in mindestens einem ersten Fermentationsbehälter (1) während einer ersten Gärstufe unter Bildung eines Fermentationsrückstandes und eines Rohbiogases;
b) Austragen des Fermentationsrückstandes aus dem ersten Fermentationsbehälter (1) und Eintragen des Fermentationsrückstandes in mindestens einen weiteren Fermentationsbehälter (2, 3) zum Nachgären des Fermentationsrückstandes in einer zweiten und/oder weiteren Gärstufe;
c) Einleiten von Wasserstoff, der mittels Elektrolyse aus Wasser und Strom erzeugt wird und einem CO₂-haltigen Gas, wobei als CO₂-haltiges Gas Rohbiogas verwendet wird, mittels mindestens einer Zuführung (8), die eintrittsseitig an eine Wasserstoffleitung (9) und eine Leitung für CO₂-haltiges Gas (4a) angeschlossen ist, in den Fermentationsrückstand während der zweiten und/oder weiteren Gärstufe und biologische Umwandlung des Wasserstoffs mittels CO₂ zu Methan.

2. Verfahren nach Anspruch 1,
**dadurch gekennzeichnet, dass** der Wasserstoff und das Rohbiogas derart in den Fermentationsrückstand eingeleitet werden, dass der Wasserstoff und das Rohbiogas durch den Fermentationsrückstand als feine Blasen strömen.

3. Verfahren nach einem der Ansprüche 1 oder 2,
**dadurch gekennzeichnet, dass** der Wasserstoff und das Rohbiogas gemischt werden und als Gasgemisch in den Fermentationsrückstand eingeleitet werden.

4. Verfahren nach einem der Ansprüche 1 bis 3,
**dadurch gekennzeichnet, dass** der Wasserstoff und das Rohbiogas mittels mindestens einer sich wenigstens teilweise über den Querschnitt des Fermentationsbehälters erstreckenden Zuführung in den Fermentationsrückstand eingeleitet werden.

5. Verfahren nach einem der Ansprüche 1 bis 4,
**dadurch gekennzeichnet, dass** das Rohbiogas in Abhängigkeit von dessen Wasserstoffgehalt in einer Kaskade in den Fermentationsrückstand während der zweiten und/oder weiteren Gärstufe geleitet wird.

6. Verfahren nach einem der Ansprüche 1 bis 5,
**dadurch gekennzeichnet, dass** das Rohbiogas am Ende der zweiten oder weiteren Gärstufe einer Aufbereitungsanlage zugeführt und mittels Entfernung von CO₂ in Bio-Erdgas umgewandelt wird.

7. Verfahren nach Anspruch 6,
**dadurch gekennzeichnet, dass** das Bio-Erdgas in ein Erdgasversorgungsnetz eingespeist wird.

8. Verfahren nach Anspruch 6,
**dadurch gekennzeichnet, dass** das Bio-Erdgas gespeichert, bedarfsweise ausgespeichert und einer Verstromungsanlage zugeführt wird.

9. Biogasanlage zum Erzeugen von Biogas,
mit mindestens einem ersten Fermentationsbehälter (1) in dem Biomasse während einer ersten Gärstufe unter Bildung eines Fermentationsrückstandes und eines Rohbiogases vergoren wird, mit mindestens einem weiteren Fermentationsbehälter (2, 3) zum Nachgären des Fermentationsrückstandes in einer zweiten und/oder weiteren Gärstufe und
mit mindestens einer Zuführung (8), die eintrittsseitig an eine Wasserstoffleitung (9) und eine Leitung für CO₂-haltiges Gas (4a) angeschlossen ist, wobei die Wasserstoffleitung (9) an eine Wasserelektrolyse (12) angeschlossen ist, wobei durch die Zuführung (8) ausgangsseitig Wasserstoff und Rohbiogas in den Fermentationsrückstand in der zweiten und/oder weiteren Gärstufe eingeleitet wird, wobei der Wasserstoff mittels Kohlenstoffdioxid biologisch zu Methan umgewandelt wird.

10. Anlage nach Anspruch 9,
**dadurch gekennzeichnet, dass** sich die Zuführung (8) in Bodennähe des Fermentationsbehälters (2, 3) erstreckt.

11. Anlage nach Anspruch 9,
**dadurch gekennzeichnet, dass** die Zuführung (8) als mindestens eine Lanze ausgebildet ist, die sich wenigstens teilweise über den Querschnitt des Fermentationsbehälters (2, 3) erstreckt.

12. Anlage nach Anspruch 9,
**dadurch gekennzeichnet, dass** die Zuführung (8) als mindestens eine konzentrische Ringleitung ausgebildet ist.

13. Anlage nach Anspruch 9,
**dadurch gekennzeichnet, dass** die Zuführung (8) als Hohlboden des Fermentationsbehälters (2, 3) ausgebildet ist.

14. Anlage nach einem der Ansprüche 9 bis 13,
**dadurch gekennzeichnet, dass** die Zuführung (8) eine Vielzahl von Durchtrittsöffnungen (11) in Form einer Gitter- oder Lochstruktur aufweist.

15. Anlage nach einem der Ansprüche 9 bis 14,
**dadurch gekennzeichnet, dass** die Zuführung (8) wenigstens teilweise als Membran ausgebildet ist.

16. Anlage nach einem der Ansprüche 9 bis 15,
**dadurch gekennzeichnet, dass** die Zuführung (8) einen Schieber (13) zum Entfernen von Sedimenten von den Durchtrittsöffhungen (11) aufweist.

## Claims

1. Method for producing biogas in a biogas plant comprising the following steps:
a) Fermentation of a biomass in at least one first fermentation tank (1) during a first fermentation stage to form a fermentation residue and a raw biogas;
b) Discharge of the fermentation residue from the first fermentation tank (1) and introduction of the fermentation residue into at least one further fermentation tank (2, 3) for post-fermentation of the fermentation residue in a second and/or further fermentation stage;
c) Introduction of hydrogen, which is produced by electrolysis from water and electricity, and a CO₂-containing gas, with the CO₂-containing gas used being raw biogas, into the fermentation residue by means of at least one feed (8), which is connected on the inlet side to a hydrogen line (9) and a line for CO₂-containing gas (4a), during the second and/or further fermentation stage and biological conversion of the hydrogen by means of CO₂ to methane.

2. Method according to claim 1,
**characterised in that** the hydrogen and the raw biogas are introduced into the fermentation residue such that the hydrogen and the raw biogas flow through the fermentation residue as fine bubbles.

3. Method according to claim 1 or 2,
**characterised in that** the hydrogen and the raw biogas are mixed and introduced as a gas mixture into the fermentation residue.

4. Method according to any of claims 1 to 3,
**characterised in that** the hydrogen and the raw biogas are introduced into the fermentation residue by means of at least one feeder extending at least partially across the cross-section of the fermentation tank.

5. Method according to any of claims 1 to 4,
**characterised in that** the raw biogas is passed in a cascade into the fermentation residue during the second and/or further fermentation stage as a function of the hydrogen content of the raw biogas.

6. Method according to one of claims 1 to 5,
**characterised in that** the raw biogas is fed to a treatment plant at the end of the second or further fermentation stage and is converted into bio natural gas by the removal of CO₂.

7. Method according to claim 6,
**characterised in that** the bio natural gas is fed into a natural gas supply network.

8. Method according to claim 6,
**characterised in that** the bio natural gas is stored, withdrawn from storage as necessary, and supplied to an electricity generation plant.

9. Biogas plant for the production of biogas,
having at least one first fermentation tank (1) in which biomass is fermented during a first fermentation stage to form a fermentation residue and a raw biogas, having at least one further fermentation tank (2, 3) for post-fermentation of the fermentation residue in a second and/or further fermentation stage, and
having at least one feeder (8) which is connected on the inlet side to a hydrogen line (9) and a line for CO₂-containing gas (4a), with the hydrogen line (9) being connected to a water electrolysis system (12), with hydrogen and raw biogas being introduced on the outlet side by the feeder (8) into the fermentation residue in the second and/or further fermentation stage, with the hydrogen being biologically converted to methane by means of carbon dioxide.

10. Plant according to claim 9,
**characterised in that** the feeder (8) extends near the bottom of the fermentation tank (2, 3).

11. Plant according to claim 9,
**characterised in that** the feeder (8) is formed as at least one lance extending at least partially across the cross-section of the fermentation tank (2, 3).

12. Plant according to claim 9,
**characterised in that** the feeder (8) is formed as at least one concentric ring line.

13. Plant according to claim 9,
**characterised in that** the feeder (8) is formed as a hollow floor of the fermentation tank (2, 3).

14. Plant according to one of claims 9 to 13,
**characterised in that** the feeder (8) exhibits a plurality of passage openings (11) in the form of a grid or hole structure.

15. Plant according to one of claims 9 to 14,
**characterised in that** the feeder (8) is formed at least partially as a membrane.

16. Plant according to one of claims 9 to 15,
**characterised in that** the feeder (8) comprises a scraper (13) for removing sediment from the passage openings (11).

## Revendications

1. Procédé de production de biogaz dans une installation de biogaz, comportant les étapes suivantes :
a) fermentation d'une biomasse dans au moins une première cuve de fermentation (1) pendant une première étape de fermentation avec formation d'un résidu de fermentation et d'un biogaz brut ;
b) déchargement du résidu de fermentation de la première cuve de fermentation (1) et chargement du résidu de fermentation dans au moins une autre cuve de fermentation (2, 3) pour la post-fermentation du résidu de fermentation dans une deuxième et/ou autre étape de fermentation ;
c) introduction d'hydrogène, qui est produit par électrolyse à partir d'eau et d'électricité, et d'un gaz contenant du CO₂, du biogaz brut étant utilisé comme gaz contenant du CO₂, dans le résidu de fermentation au moyen d'au moins un dispositif d'amenée (8) qui est raccordé du côté entrée à une conduite d'hydrogène (9) et à une conduite de gaz contenant du CO₂ (4a), pendant la deuxième et/ou autre étape de fermentation et transformation biologique de l'hydrogène en méthane au moyen du CO₂.

2. Procédé selon la revendication 1,
**caractérisé en ce que** l'hydrogène et le biogaz brut sont introduits dans le résidu de fermentation de telle sorte que l'hydrogène et le biogaz brut s'écoulent en fines bulles à travers le résidu de fermentation.

3. Procédé selon l'une des revendications 1 ou 2,
**caractérisé en ce que** l'hydrogène et le biogaz brut sont mélangés et introduits sous forme de mélange de gaz dans le résidu de fermentation.

4. Procédé selon l'une des revendications 1 à 3,
**caractérisé en ce que** l'hydrogène et le biogaz brut sont introduits dans le résidu de fermentation au moyen d'au moins un dispositif d'amenée s'étendant au moins partiellement sur la section transversale de la cuve de fermentation.

5. Procédé selon l'une des revendications 1 à 4,
**caractérisé en ce que** le biogaz brut est introduit en cascade en fonction de sa teneur en hydrogène dans le résidu de fermentation pendant la deuxième et/ou autre étape de fermentation.

6. Procédé selon l'une des revendications 1 à 5,
**caractérisé en ce que** le biogaz brut est amené à une installation de traitement à la fin de la deuxième ou autre étape de fermentation et est transformé en gaz naturel biologique par élimination du CO₂.

7. Procédé selon la revendication 6,
**caractérisé en ce que** le gaz naturel biologique est injecté dans un réseau d'alimentation en gaz naturel.

8. Procédé selon la revendication 6,
**caractérisé en ce que** le gaz naturel biologique est stocké, si nécessaire déstocké et amené à une installation de production d'électricité.

9. Installation de biogaz pour la production de biogaz,
comportant au moins une première cuve de fermentation (1) dans laquelle une biomasse est mise à fermenter pendant une première étape de fermentation avec formation d'un résidu de fermentation et d'un biogaz brut, avec au moins une autre cuve de fermentation (2, 3) pour la post-fermentation du résidu de fermentation dans une deuxième et/ou une autre étape de fermentation et
comportant au moins un dispositif d'amenée (8) qui est raccordé du côté entrée à une conduite d'hydrogène (9) et à une conduite de gaz contenant du CO₂ (4a), la conduite d'hydrogène (9) étant raccordée à une électrolyse d'eau (12), de l'hydrogène et du biogaz brut étant introduits par le dispositif d'amenée (8) du côté sortie dans le résidu de fermentation dans la deuxième et/ou autre étape de fermentation, l'hydrogène étant transformé biologiquement en méthane au moyen du dioxyde de carbone.

10. Installation selon la revendication 9,
**caractérisée en ce que** le dispositif d'amenée (8) s'étend à proximité du fond de la cuve de fermentation (2, 3).

11. Installation selon la revendication 9,
**caractérisée en ce que** le dispositif d'amenée (8) est réalisé sous la forme d'au moins une lance qui s'étend au moins partiellement sur la section transversale de la cuve de fermentation (2, 3).

12. Installation selon la revendication 9,
**caractérisée en ce que** le dispositif d'amenée (8) est réalisé sous la forme d'au moins une conduite annulaire concentrique.

13. Installation selon la revendication 9,
**caractérisée en ce que** le dispositif d'amenée (8) est réalisé sous la forme d'un fond creux de la cuve de fermentation (2, 3).

14. Installation selon l'une des revendications 9 à 13,
**caractérisée en ce que** le dispositif d'amenée (8) présente une pluralité d'ouvertures de passage (11) sous la forme d'une structure en grille ou à trous.

15. Installation selon l'une des revendications 9 à 14,
**caractérisée en ce que** le dispositif d'amenée (8) est réalisé au moins partiellement sous la forme d'une membrane.

16. Installation selon l'une des revendications 9 à 15,
**caractérisée en ce que** le dispositif d'amenée (8) présente un poussoir (13) pour éliminer les sédiments des ouvertures de passage (11).
